# EUROPÄISCHE PATENTSCHRIFT

(11) **EP 1 242 350 B1**
(45) Veröffentlichungstag und Bekanntmachung des Hinweises auf die Patenterteilung: **31.03.2004**
(21) Anmeldenummer: 00990667.8
(22) Anmeldetag: 07.12.2000
(51) Int. Cl.: C07C 39/16, C07C 37/70

(54) **BISPHENOL-PHENOL-ADDUKTE**
BISPHENOL PHENOL ADDUCTS
ADDUITS BISPHENOL-PHENOL

(30) Priorität: 20.12.1999 DE 19961521
(43) Veröffentlichungstag der Anmeldung: 25.09.2002
(73) Patentinhaber: Bayer MaterialScience AG, 51368 Leverkusen (DE)
(72) Erfinder: NEUMANN, Rainer, 47803 Krefeld (DE); LANZE, Rolf, 47804 Krefeld (DE); HEYDENREICH, Frieder, 40593 Düsseldorf (DE); BÖDIGER, Michael, League City, TX 77573 (US); PREIN, Michael, B-2930 Brasschaat (BE)
(86) Internationale Anmeldenummer: PCT/EP2000/012323
(87) Internationale Veröffentlichungsnummer: WO 2001/046105

(56) Entgegenhaltungen:
- EP-A- 0 926 118

## Beschreibung

Die vorliegende Erfindung betrifft ein Verfahren zur Herstellung von Bis(4-hydroxyaryl)alkanen hoher Reinheit aus Addukten von Bis(4-hydroxyaryl)alkanen und aromatischen Hydroxyverbindungen, die durch sauer katalysierte Umsetzung der aromatischen Hydroxyverbindungen mit Ketonen erhalten werden.

Bisphenole als Kondensationsprodukte von Phenolen und Carbonylverbindungen sind Ausgangsstoffe oder Zwischenprodukte zur Herstellung einer Vielzahl kommerzieller Produkte. Von besonderer technischer Bedeutung ist das Kondensationsprodukt aus der Reaktion zwischen Phenol und Aceton, 2,2-Bis(4-hydroxyphenyl)-propan (BPA). BPA dient als Ausgangstoff zur Herstellung verschiedenartiger polymerer Werkstoffe wie beispielsweise Polyarylate, Polyetherimide, Polysulfone und modifizierter Phenol-Formaldehydharze. Bevorzugte Anwendungsgebiete liegen in der Herstellung von Epoxyharzen und Polycarbonaten.

Technisch relevante Herstellmethoden für BPA sind bekannt und beruhen auf der säurekatalysierten Umsetzung von Phenol mit Aceton, wobei ein bevorzugt Phenol-Aceton-Verhältnis von größer 5 : 1 in der Reaktion eingestellt wird. Als saure Katalysatoren können homogene wie auch heterogene Brönsted- oder Lewissäuren genutzt werden, so beispielsweise starke Mineralsäuren wie Salz- oder Schwefelsäure. Bevorzugt kommen gelförmige oder makroporöse sulfonierte vernetzte Polystyrolharze (saure Ionentauscher) zum Einsatz.

Bei der Umsetzung von Phenol mit Aceton in Gegenwart saurer Katalysatoren entsteht eine Produktmischung, die neben nicht umgesetzten Phenol und gegebenenfalls Aceton in erster Linie BPA und Wasser enthält. Daneben treten in geringen Mengen typische Nebenprodukte der Kondensationreaktion auf, so beispielsweise 2-(4-hydroxyphenyl)-2-(2-hydroxyphenyl)propan (o,p-BPA), substituierte Indene, Hydroxyphenyl-indanole, Hydroxyphenyl-chromane, substituierte Xanthene und höher kondensierte Verbindungen mit drei oder mehr Phenylringen im Molekülgerüst.

Die genannten Nebenprodukte wie auch Wasser, Phenol und Aceton beeinträchtigen die Eignung von BPA zur Herstellung von Polymeren und müssen durch geeignete Verfahren abgetrennt werden. Insbesondere zur Herstellung von Polycarbonat werden hohe Reinheitsanforderungen an den Rohstoff BPA gestellt.

Eine Aufarbeitungs- und Reingungsmethode von BPA erfolgt durch Abtrennung von BPA aus der Reaktionsmischung in Form eines etwa äquimolaren kristallinen Addukts mit Phenol durch Abkühlen der Reaktionsmischung unter Auskristallisieren des BPA/Phenol-Addukts als Suspensionskristallisation. Die BPA/Phenol-Adduktkristalle werden anschließend durch eine geeignete Apparatur zur Fest-Flüssigtrennung wie Drehfilter oder Zentrifugen von der Flüssigphase abgetrennt und der weiteren Reinigung zugeführt (Siehe zum Beispiel EP-A-0 926 118). Dabei gelangt die aus den Kristallern austretende Kristallmaische z.B. zu den Drehfiltern und wird der Wanne des Drehfilters zugeführt, die Drehfiltertrommel taucht in die darin vorhandene Kristallmaische ein.

Die Drehfiltertrommel ist über einen Steuerkopf in unterschiedliche Segmente aufgeteilt. An die Ansaugzone, welche in den Bereich der Drehfilterwanne gelegt ist, schließt sich eine Waschzone an, in der der Filterkuchen durch Aufsprühen von Phenol über Düsenreihen gewaschen wird, gefolgt von einer Trockenzone, in welcher restliches im Filterkuchen enthaltenes Phenol soweit möglich abgesaugt wird. Ansaugzone, Waschzone und Trockenzone sind jeweils mit den zugehörigen Vakuumaggregaten verbunden.

Anschließend an die Trockenzone wird über eine Entspannungszone im Steuerkopf ein Druckausgleich zwischen Saugkammer und Drehfiltergehäuse durchgeführt, um ein problemloses Abfallen des Filterkuchens beim Erreichen der Schaberabnahme zu ermöglichen. Das Filtertuch wird nach der Abnahme des Filterkuchens von Eintritt in die Drehfilterwanne über eine Düsenreihe mit Phenol gewaschen.

Für ein optimales Verfahren mit hoher Ausbeute und hoher Reinheit zur Herstellung von BPA ist die Abtrennung der durch Kristallisation gewonnenen BPA-Phenol-Adduktkristalle von der Mutterlauge, die einen Großteil der Verunreinigungen enthält, und die Reinigung der Adduktristalle von entscheidender Bedeutung.

Aufgabe ist die Bereitstellung eines Verfahrens zur optimalen Abtrennung der BPA-Phenol-Adduktkristalle von der Mutterlauge sowie die Reinigung der Kristalle.

Es wurde nun gefunden, dass durch ein spezielles Abtrennungs- und Reinigungsverfahren BPA wirtschaflich und effektiv erhalten wird.

Die nachfolgenden Ausführungen beziehen sich im wesentlichen auf die oben beschriebenen BPA Herstellungsverfahren.

Gegenstand der Erfindung ist ein Verfahren zur Abtrennung und Reinigung von bei der Herstellung durch sauer katalysierte Umsetzung von aromatischen Hydroxyverbindungen mit Ketonen erhalten Adukkten von Bis(4-hydroxyaryl)alkanen und aromatischen Hydroxyverbindungen, das dadurch gekennzeichnet ist, dass die im Prozess gebildeten Adduktkristalle nach der Kristallisation durch kontinuierliche Filtration aus der Suspension in einem mehrere Filterzellen enthaltenden und rotierenden Vakuumtrommelfilter von der Mutterlauge getrennt, anschließend gewaschen und die Waschflüssigkeit abgesaugt wird.

Vorzugsweise betrifft die Erfindung die Abtrennung und Reinigung der bei der Herstellung durch saurer katalysierte Umsetzung von Phenol und Aceton anfallenden 2,2-Bis(4-hydroxyphenyl)propan-Phenol-Adduktkristallen (BPA-Phenol-Adduktkristalle).

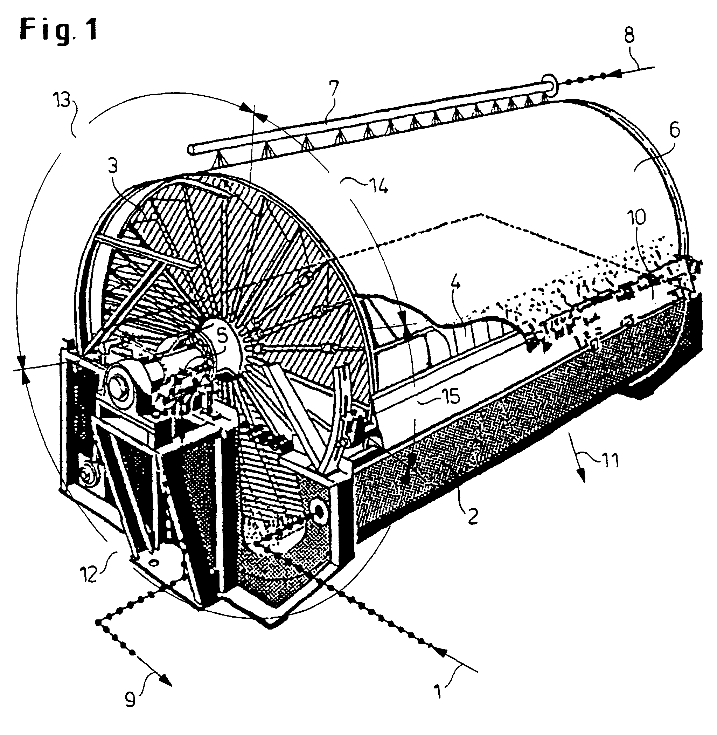

Die Vakuumtrommelfilter sind bekannt (z.B. Krauss Maffei Trommelfilter TSF; Fig.1). Vorzugsweise enthalten die Trommelfilter als Filterzellen eine Ansaugzone (12), eine Waschzone (13), eine Trockensaugzone (14), eine Belüftungszone (15) und gegebenenfalls eine Kuchenabnahmezone (10) und eine Tuchspülungszone.

Über einen Zulaufstrom gelangt die bei der Kristallisation anfallende BPA-Phenol-Adduktkristall-Suspension in den Vakuumtrommelfilter. Der Feststoffanteil im Zulaufstrom liegt vorzugsweise bei 5 - 35%, insbesondere bei 20 - 30%.

Der Zulaufstrom hat vorzugsweise eine Temperatur von 40 bis 70°C, insbesondere von 40 bis 45° C, bevorzugt um die 41°C.

Die Menge der Adduktkristalle die pro Zeiteinheit und m²-Filterfläche von der Suspensionsflüssigkeit als Filterkuchen abgetrennt werden liegt vorzugsweise bei 100 bis 800 kg/h, insbesondere bei 300 bis 700 kg/h.

In der Ansaugzone wird vorzugsweise ein Vakuum von 5 bis 500 mbar angelegt.

In der Waschzone wird vorzugsweise ein Vakuum von 5 bis 300 mbar angelegt.

In der Trockensaugzone wird vorzugsweise ein Vakuum von 5 bis 500 mbar angelegt.

Die Spülung des Filterkuchens in der Waschzone wird mit reinem Phenol bei Temperaturen von vorzugsweise 45 bis 70°C, insbesondere 50 bis 60°C durchgeführt.

Die Filterkuchenreinigung wird vorzugsweise mit einer Spülmenge von 50 - 150%, bezogen auf die Filterkuchenmenge durchgeführt.

Die Wasch- bzw. Spülflüssigkeit wird über Waschdüsen, vorzugsweise 10 bis 30 Waschdüsen eingebracht. Die Düsen sind im allgemeinen vorzugsweise so angeordnet, dass deren Sprühkegel sich auf dem Filterkuchen überlappen.

Die Tuchspülung wird vorzugsweise mit reinem oder aus dem Prozess durch Destillation zurückgewonnenem Phenol bei Temperaturen von 70 bis 85°C, vorzugsweise 78 bis 82°C durchgeführt.

Die Tuchspülung wird vorzugsweise miteiner Spülmenge von 20 bis 100%, bezogen auf die Menge des Filterkuchens durchgeführt.

Als Filtertuch wird ein phenol- und temperaturbeständiges Filtertuch, vorzugsweise 1- oder 2fach kalandriert, mit einer Luftdurchlässigkeit von vorzugsweise 300 bis 1500 l/dm²/min, insbesondere 500 - 900 l/dm²/min, eingesetzt.

Das Drehfiltergehäuse wird vorzugsweise unter einem leichtem Überdruck von 20 mbar, bevorzugt 10 mbar mit Sticktoff inertisiert. Auch der gewaschene Filterkuchen wird vorzugsweise mit Stickstoff durchströmt. Der eingesetzte Stickstoff hat vorzugsweise einen Sauerstoffgehalt von weniger als 1ppm und wird vorzugsweise im Kreislauf geführt wobei vorzugsweise etwa 3-10% der Kreislaufmenge kontinuierliche ausgespeisst und durch Reinstickstoff ersetzt wird. Vorzugsweise wird der Kreislaufstickstoff mit VE-Wasser gewaschen.

Die Trommeldrehzahl, Filterkuchendicke, Kreislaufstickstoffmenge und die Ansaugöffnungen der Steuerscheibe wird so eingestellt, dass die Restfeuchte im Filterkuchen unter 30%, vorzugsweise 20% insbesondere unter 15 %, bezogen auf die Mischkristallmenge, liegt.

Trommelrundheit und Schälmesserausrichtung wird so eingestellt, dass ein maximaler Schälmesserabstand zur Trommel von vorzugsweise 4 - 6 mm über die gesamte Filterfläche anliegt.

In einer bevorzugten Ausführungsform werden die so erhaltenen erfindungsgemäß abgetrennten und gereinigten BPA-Phenol-Adduktkristalle auf einer Heizspirale aufgeschmolzen und, um die Kontaktzeit der Addukkristalle auf der heißen Edelstahloberfläche (1.4571) auf ein Minimum zu begrenzen, unmittelbar als Schmelze in einen Auffangbehälter abfließen gelassen.

Die Oberflächentemperatur der Heizspirale beträgt vorzugsweise 130 bis 230°C, bevorzugt 150 bis 170°C. Die Aufschmelzung wird vorzugsweise unter inerten Bedingungen, insbesondere unter Sauerstoffausschluss (Sauerstoffgehalt < 1ppm) durchgeführt.

In Fig. 1 bedeuten:

| | |
|---|---|
| 1) Mutterlaugenzuführung | 9) Mutterlaugenablauf |
| | |
| 2) Wanne | 10) Schaberabnahme |
| | |
| 3) Filterzone | 11) Feststoff |
| | |
| 4) Filtertuch | 12) Ansaugzone |
| | |
| 5) Steuerkopf | 13) Waschzone |
| | |
| 6) Filterkuchen | 14) Trockenzone |
| | |
| 7) Waschdüse | 15) Belüftungszone |
| | |
| 8) Waschflüssigkeit | |

Die nachfolgenden Beispiele dienen zur Erläuterung der Erfindung. Die Erfindung ist nicht auf die Beispiele limitiert. Wenn nichts anderes angegeben stehen Prozentangaben für Gewichtsprozente.

### Beispiele

### Beispiel 1

Die bei der säurekatalysierten Umsetzung von Phenol und Aceton mit anschließender Suspensionskristallisation anfallenden BPA/Phenol-Adduktkristalle werden durch ein Drehfilter von der Flüssigphase abgetrennt und der weiteren Reinigung zugeführt. Hierzu wird ein Feststoffanteil im Zulaufstrom von 25%, eine Zulauftemperatur von 41°C, sowie eine Menge der abgetrennten Mischkristalle (Filterkuchen) pro m²-Filterfläche von 500 kg/h eingestellt.

Filtriert wird auf einem phenol- und temperaturbeständigen Filtertuch (2fach kalandriert) mit einer Luftdurchlässigkeit von 700 l/dm²/min. Die Vakua in der Ansaugzone betragen 100 mbar, in der Waschzone 80 mbar und in der Trockensaugzone 100 mbar. Das Drehfiltergehäuse wird dabei unter einem leichtem Überdruck von 10 mbar mit Sticktoff (< 1ppm Sauerstoff) inertisiert.

Trommeldrehzahl, Filterkuchendicke, Kreislaufstickstoffmenge (Sauersoffgehalt < 1 ppm) und die Ansaugöffnungen der Steuerscheibe sind so eingestellt, dass die Restfeuchte im Filterkuchen < 15 % bezogen auf die Mischkristallmenge beträgt. Weiterhin erlaubt die Trommelrundheit und Schälmesserausrichtung einen maximalen Schälmesserabstand zur Trommel von etwa 5 mm über die gesamte Filterfläche

Für die Spülung des Filterkuchens in der Waschzone wird reines Phenol mit einer Temperatur von 55°C eingesetzt, wobei die Spülmenge für die Filterkuchenreinigung 100 %, bezogen auf die Filterkuchenmenge, beträgt. Zur optimalen Filterkuchenwäsche werden 20 Waschdüsen eingesetzt, wobei die Düsen so angeordnet werden, dass deren Sprühkegel sich auf dem Filterkuchen überlappen. Der gewaschene Filterkuchen wird mit Stickstoff (Sauerstoffgehalt < 1ppm) durchströmt, der im Kreislauf geführt wird, wobei etwa 7% der Kreislaufmenge kontinuierlich ausgespeist und durch Reinstickstoff ersetzt werden und der Kreislaufstickstoff mit VE-Wasser gewaschen wird.

Unmittelbar nach der Kuchenabnahme werden die Mischkristalle auf einer Heizspirale unter inerten Bedingungen (Sauerstoffgehalt < 1ppm) aufgeschmolzen, die Schmelze fließt sofort in eine Auffangbehälter ab, um die Kontaktzeit der Kristalle auf der heißen Edelstahloberfläche (1.4571) auf ein Minimum zu begrenzen. Die Oberflächentemperatur der Heizspirale beträgt etwa 160°C.

Für die Tuchspülung wird Phenol mit einer Temperatur 80°C eingesetzt, wobei die Spülmenge zur Tuchspülung, bezogen auf die Menge des Filterkuchens, 80% beträgt.

Durch diese Art der Filtration wird ein BPA-Phenoladdukt mit hohen Reinheiten (>99% ohne Phenolanteil) erhalten.

## Patentansprüche

1. Verfahren zur Abtrennung und Reinigung von bei der Herstellung durch sauer katalysierte Umsetzung von aromatischen Hydroxyverbindungen mit Ketonen erhalten Adukkten von Bis(4-hydroxyaryl)alkanen und aromatischen Hydroxyverbindungen, das **dadurch gekennzeichnet ist, dass** die im Prozess gebildeten Adduktkristalle nach der Kristallisation durch kontinuierliche Filtration aus der Suspension in einem mehrere Filterzellen enthaltenden und rotierenden Vakuumtrommelfilter von der Mutterlauge getrennt, anschließend gewaschen und die Waschflüssigkeit abgesaugt wird.

2. Verfahren gemäß Anspruch 1, **dadurch gekennzeichnet, dass** 2,2-Bis(4-hydroxyphenyl)propan-Phenol-Adduktkristallen (BPA-Phenol-Adduktkristalle) die bei der sauer katalysierten Umsetzung von Phenol mit Aceton anfallenden, abgetrennt und gereinigt werden.

3. Verfahren gemäß Anspruch 1, **dadurch gekennzeichnet, dass** die Trommelfilter als Filterzellen eine Ansaugzone, eine Waschzone, eine Trockensaugzone, eine Belüftungszone und gegebenenfalls eine Kuchenabnahmezone und eine Tuchspülungszone enthalten.

4. Verfahren gemäß Anspruch 1, **dadurch gekennzeichnet, dass** die Menge der Adduktkristalle die pro Zeiteinheit und m²-Filterfläche von der Suspensionsflüssigkeit als Filterkuchen abgetrennt werden bei 100 bis 800 kg/h liegt.

5. Verfahren gemäß Anspruch 1, **dadurch gekennzeichnet, dass** die Spülung des Filterkuchens in der Waschzone mit reinem Phenol bei Temperaturen von 45 bis 70° C und mit einer Spülmenge von 50 - 150%, bezogen auf die Filterkuchenmenge, durchgeführt wird.

6. Verfahren gemäß Anspruch 1, **dadurch gekennzeichnet, dass** die Trommeldrehzahl, Filterkuchendicke, Kreislaufstickstoffmenge und die Ansaugöffnungen der Steuerscheibe so eingestellt wird, dass die Restfeuchte im Filterkuchen unter 30% liegt.

7. Verfahren gemäß Anspruch 1, **dadurch gekennzeichnet, dass** die gereinigten BPA-Phenol-Adduktkristalle auf einer Heizspirale aufgeschmolzen werden und unmittelbar als Schmelze in einen Auffangbehälter abfließen.

8. Verfahren gemäß einem der Ansprüche 2-7, **dadurch gekennzeichnet, dass** 2,2-Bis(4-hydroxyphenyl)propan eine Reinheit von >99% aufweist.

## Claims

1. A process for separating and purifying adducts of bis(4-hydroxyaryl)alkanes and aromatic hydroxy compounds obtained during production by acid-catalysed reaction of aromatic hydroxy compounds with ketones, which process is **characterised in that** the adduct crystals formed in the process are separated from the mother liquor after crystallisation out of the suspension by continuous filtration in a revolving vacuum drum filter containing a plurality of filter cells and then washed, and the washing liquid is drawn off.

2. A process according to claim 1, **characterised in that** 2,2-bis(4-hydroxyphenyl)propane/phenol adduct crystals (BPA/phenol adduct crystals) arising during acid-catalysed reaction of phenol and acetone are separated off and purified.

3. A process according to claim 1, **characterised in that** the drum filters contain as filter cells a cake-forming zone, a washing zone, a drying suction zone, an aeration zone and optionally a cake removal zone and a cloth rinsing zone.

4. A process according to claim 1, **characterised in that** the quantity of adduct crystals separated from the suspension liquid as filter cake per unit time and per m² filter surface area is 100 to 800 kg/h.

5. A process according to claim 1, **characterised in that** filter cake rinsing is performed in the washing zone using pure phenol at temperatures of from 45 to 70 °C and using a rinsing agent quantity of 50 - 150 % relative to the quantity of filter cake.

6. A process according to claim 1, **characterised in that** the drum rotation speed, filter cake thickness, recirculated nitrogen quantity and the suction openings in the control disk are set such that the residual moisture in the filter cake is below 30 %.

7. A process according to claim 1, **characterised in that** the purified BPA/phenol adduct crystals are melted on a heating coil and flow directly into a receiving tank as a melt.

8. A process according to one of claims 2 - 7, **characterised in that** 2,2-bis(4-hydroxyphenyl)propane has a purity of >99%.

## Revendications

1. Procédé de séparation et d'épuration de produits d'addition de bis(4-hydroxyaryl)alcanes et de composés hydroxylés aromatiques obtenus lors de la fabrication par conversion catalytique acide de composés hydroxylés aromatiques avec des cétones, qui est **caractérisé en ce que** , après la cristallisation, les cristaux de produit d'addition formés lors du processus à partir de la suspension sont séparés de la liqueur-mère par filtration continue dans un filtre à vide à tambour rotatif comportant plusieurs cellules de filtration, ensuite lavés et **en ce que** le liquide de lavage est aspiré.

2. Procédé suivant la revendication 1, **caractérisé en ce que** des cristaux de produit d'addition de 2,2-bis(4-hydroxyphényl)propane-phénol (cristaux de produit d'addition de BPA-phénol) obtenus lors de la fabrication par conversion catalytique acide de phénol et d'acétone sont séparés et épurés.

3. Procédé suivant la revendication 1, **caractérisé en ce que** les filtres à tambour contiennent comme cellules de filtration une zone d'aspiration, une zone de lavage, une zone de séchage, une zone de ventilation et le cas échéant une zone d'enlèvement du gâteau de filtration et une zone de rinçage de la toile de filtration.

4. Procédé suivant la revendication 1, **caractérisé en ce que** la quantité de cristaux de produits d'addition qui peut être séparée sous forme de gâteau de filtration du liquide de la suspension par unité de temps et m² de surface de filtration se situe entre 100 et 800 kg/h.

5. Procédé suivant la revendication 1, **caractérisé en ce que** le lavage du gâteau de filtration dans la zone de lavage est effectué avec du phénol pur à des températures de 45 à 70° C et avec une quantité de rinçage de 50 - 150%, rapportée à la quantité de gâteau de filtration.

6. Procédé suivant la revendication 1, **caractérisé en ce que** la vitesse de rotation du tambour, l'épaisseur du gâteau de filtration, le débit d'azote en circulation et les ouvertures d'aspiration du disque de commande sont réglés de telle façon que l'humidité résiduelle dans le gâteau de filtration est inférieure à 30%.

7. Procédé suivant la revendication 1, **caractérisé en ce que** les cristaux de produit d'addition de BPA-phénol épurés sont fondus sur un serpentin de chauffage et coulent immédiatement comme matière fondue dans un récipient de collecte.

8. Procédé suivant l'une des revendications 2-7, **caractérisé en ce que** le 2,2-bis(4-hydroxyphényl)propane présente une pureté > 99%.
